# EUROPEAN PATENT APPLICATION

(11) **EP 2 116 266 A1**
(43) Date of publication of application: **11.11.2009**
(21) Application number: 08711954.1
(22) Date of filing: 26.02.2008
(51) Int. Cl.: A61K 47/30, A61K 9/70, A61K 45/00, A61K 47/32, A61K 47/34, A61K 47/36, A61P 43/00

(54) **PERCUTANEOUSLY ABSORBABLE PREPARATION, PROCESS FOR PRODUCTION THEREOF AND METHOD FOR PERCUTANEOUS ABSORPTION**

(30) Priority: 01.03.2007 JP 2007089369
(71) Applicant: Cosmed Pharmaceutical Co., Ltd., Kamigyo-ku Kyoto-shi Kyoto 602-0841 (JP)
(72) Inventor: KAMIYAMA, Fumio, Kyoto-shi Kyoto 602-0841 (JP); QUAN, Ying-shu, Kyoto-shi Kyoto 602-0841 (JP)
(74) Representative: Merkle, Gebhard
(86) International application number: PCT/JP2008/053225
(87) International publication number: WO 2008/108209

(57) **Abstract**

This invention is to provide a transdermal permeation preparation which includes a concentrated transdermal active ingredient laminate and exhibits high transdermal permeation effect; and a method for producing the preparation

A transdermal permeation preparation which comprises a base material which is a hydrophilic macromolecule which can swell after absorbing the water and/or water-containing alcohol and comprises a transdermal active ingredient which is in a molecular weight higher than 1000 Dalton and can dissolve or suspend in water and/or water-containing alcohol laminated on the surface of the base material, ; and a method for producing the transdermal permeation preparation wherein the transdermal active ingredient of a molecular weigh higher than 1000 Dalton is dissolved or suspended in water and/or water-containing alcohol to form an aqueous solution or suspension and the said aqueous solution or suspension is coated on the surface of base materials which are hydrophilic macromolecules which can swell after absorbing the water or e water-containing alcohol as a result of absorption of the water or the water-containing alcohol in the base material, thereby forming a layer containing the transdermal active ingredient in a concentrated state on the surface of the base material.

## Description

### Field of invention

The present invention relates to a transdermal permeation preparation, method for producing the preparation, and transdermal permeation method using the preparation.

### Background of the invention

It is known that transdermal preparation (topical dermatological preparation), such as lotion, ointment, cream, cataplasm, and tape etc. is added with some active ingredients to perform the therapeutic effect percutaneously. However, both the insufficient drug concentration in base material of the transdermal preparation and the function of skin to protect the body from external insults make it difficult to obtain a safficient transdermal permeation and a therapeutic effect, especially in case of macromolecule being the transdermal active ingredient.

In order to increase the transdermal permeation of the transdermal active ingredients, permeation enhancers, such as nonproton solvent of dimethylsulphoxide, N,N-dimethylformamide etc. (see Patent 1), 1-dodecylazacycloheptan-2-one (see Patent 2), 1-carbon, menthone, piperitone (see Patent 3), d-limonene (see Patent 4) and lauric acid diethanolamide, are included into the matrix of the transdermal administration preparations.

However, said transdermal enhancers can not totally satisfy the requirements for drug transdermal enhancement, safety of skin irritation and lacking of unpleasant odor. Therefore, a transdermal enhancer of facilitating usage and high percutaneous enhancing effect is expected to be developed. Although some physiochemical methods, such as iontophoresis, electroporation and sonophoresis have been proposed for active transfer of drugs through the skin, the demerits of skin irritation and skin damnification make them not to be satisfying strategies.
[Patent 1] U.S. patent 3,551,554.
[Patent 2] Japanese Patent Laid-Open No. 52-1035
[Patent 3] Japan Patent Laid-Open No. 2-193932.
[Patent 4] Japan Patent Laid-Open No. 2-207024.
[Patent 5] Japan Patent Laid-Open No. 2001-58961.

### Summary of the invention

An object of this invention is to provide a transdermal preparation which can overcome said deficiency and exhibit high transdermal permeation activity containing a high concentration transdermal active ingredient; a method for producing the preparation; and a transdermal permeation method using the preparation.

The transdermal preparation in this invention is characterized by the laminate of water soluble or water swellable macromolecular active ingredients which have molecular weight of more than 1,000 Dalton, and hydrophilic macromolecular base materials which can absorb water or water-alcohol mixtures and swell.

Said transdermal active ingredient has a molecular weight higher than 1000 Dalton, and can dissolve or suspend in water and/or water-containing alcohol. If the molecular weight of the transdermal active ingredinet is lower than 1000 Dalton, the transdermal active ingredient, together with water and/or water-containing alcohol, would be absorbed into the base material and be difficult to form concentrated drug layer on the base material. So, it is necessary that the molecular weight of the transdermal active ingredient should be higher than 1000 Dalton. Said transdermal active ingredient should a macromolecular substance, such as biologically active peptides and their derivatives, nucleic acid, oligonucleotide and polysaccharides etc.

Said biologically active peptides and their inductors can be, for example, calcitonin, adrenocortical hormone, parathyroid hormone, hPTH (1→34), insulin, secretin, oxytocin, angiotensin, β-endorphin, glucagons, vasopressin, somatostatin, gastrin, luteinizing hormone-releasing hormone, enkephalin, neurotensin, atrial natriuretic peptide, growth hormone, Growth hormone releasing hormone, bradykinin, substance P, dynorphin, thyroid stimulating hormone, interferon, interleukin, G-CSF, Glutathione Peroxidase, superoxide dismutase, 1-deamino-8-d-arginine vasopressin, somatomedin, or their salts. Said oligonucleotide can be si-RNA etc. Said polysaccharide compounds can be hyaluronic acid, Immunopotentiating polysaccharides, etc.

The said base material is hydrophilic macromolecule which can swell after absorbing water and/or water-containing alcohol. Because said transdermal active ingredient can dissolve and/or suspend in water and/or water-containing alcohol; when said transdermal active ingredient in water and/or water-containing alcohol is applied on the surface of said base material, the water and/or the water-containing alcohol would be absorbed into the hydrophilic macromolecule base material, thereby forming a transdermal active ingredient layer of high concentration on the surface of the base material.

Said hydrophilic macromolecule can be poly-alkylalkoxy acrylic acids, starch-acrylic acid graft polymers, polyacrylates, polyvinylalcohol, vinyl acetate-acrylic acid polymers, polyvinyl pyrrolidone, Isobutylene--maleic acid polymer, N-vinyl acetamides.

If said hydrophilic macromolecule is cross linked, the mechanical strength of the macromolecule may not be weakened or be slightly weakened though the macromolecule swells after the absorbing of the water and/or water-containing alcohol. Therefore, it is desirable that the macromolecule is crosslinked. Typical method for crosslinking can be any known method, such as including crosslinking agent like poly isocyanate, metal complexes of aluminum acetylacetonate etc. into the hydrophilic macromolecule.

If the hydrophilic macromolecule is a polymer of poly-alkylalkoxy acrylate, it is preferable that it comprises copolymer, octyldodecyl lactate, glycerin and hyaluronic acid in a concentration ratio of 100: 2-15:2-10:0.01-0.1 by weight respectively. The component of said copolymer are methoxyethyl acrylate, (methyl) lauryl acrylate and polar monomer in a concentration ratio of 40-60%:30-40%:10-25% by weight.

Examples of said polar monomer are vinyl-2-pyrrolidone, (methyl) acrylic acid, 2-hydroxyehtyl acrylate etc. If the amount of methoxyethyl acrylate is added more than the said upper limit, it will cause gelation and infusibility of the copolymer, as well as reduction of the adhesiveness of the base material. Additionally, if the amount of (methyl)lauryl acrylate is added less than the said lower limit, the adhesiveness of the adhesive would also be reduced. Moreover, if the amount of polar monomer is added less than lower limit, the cohesive force of the adhesive would be reduced. However, a larger addition of the polar monomer will also influence the adhesiveness of the adhesive. Therefore, it is preferable that methoxyethyl acrylate, (methyl)lauryl acrylate and polar monomer are in a ratio as mentioned above. Octyldodecyl lactate, glycerin and hyaluronic acid can not only increase the hydrophilicity of said hydrophilic macromolecule, which results in higher water absorptivity of said macromolecule, but also improves its skin adhesiveness, making it easier to adhere to the skin.

If the hydrophilic macromolecule is a polymer of polyvinylalcohol, it is good to use polyvinylalcohol hydrogel. Typical method for producing the polyvinyl alcohol hydrogel sheet can be any known method. For example, a polyvinylalcohol aqueous solution in a concentration of 5∼50% by weight is spread onto the polyethylene terephthalate (PET) film to form a layer of polyvinylalcohol aqueous solution. After being frozen at -10°C∼-2°C for 1 day, said aqueous solution layer is thawed to room temperature, and then a cross-linked polyvinylalcohol sheet is prepared.

The transdermal permeation preparation in this invention comprises a transdermal active ingredient laminated on the surface of the base material. The thickness of the base material according to this invention is not specifically limited. Too thinner base material layer would influence the sufficient permeation of water and/or water-containing alcohol of the base material from the aqueous solution or suspension of the transdermal active ingredient. However, too thicker base material layer will lose its pliability, thereby resulting in lower skin adhesiveness. Therefore, the thickness of the base material layer is in a range of 20-5000µm, preferably 50-2000µm. The type of transdermal permeation preparation according to this invention is also not specifically limited. A patch preparation is desirable. Additionally, it is good to have a backing film to be laminated on surface of the base material and transdermal active ingredient. Examples of the backing films are release film, polyethylene, polypropylene, polyethylene terephthalate film and urethane film.

It is desirable to include a plasticizer which is soluble in said hydrophilic macromolecule into the base material to increase its permeation of water and/or water-containing alcohol. A hydrophilic and liquid plasticizer is preferable. Examples of the plasticizers are glycerin, ethylene glycol, polyethylene glycol, octyldodecyl lactate, sorbitan monooleate, sorbitan monopalmitate, polyoxyethylene sorbitan monooleate etc..

In order to promote percutaneous permeation of the transdermal active ingredient, it is desirable to include chemical enhancer(s) into the base material. Typical chemical enhancers can be any known chemical enhancers, for example, alcohols of menthol, camphor, cetyl alcohol etc., fatty acid esters of isopropyl palmitate, isopropyl myristate etc., glycerol esters of glyceryl monolaurate, glyceryl monooleate, etc., amides of Lauric diethanolamide, nonionic surfactants of polyethylene glycol di-n-dodecyl ether, etc. A less than necessary addition of the chemical enhancer would not obtain the enhancing effect. However, an overdose of the chemical enhancer would reduce the adhesiveness of the base material. Thus, it is preferable that the base material and chemical enhancer are mixed in a ratio of 100:3-40 by weight.

If some enzyme as protease, esterase, lipase etc. are to be included into the base material, the percutaneous permeation of transdermal active ingredient can be much more enhanced due to the effect of these enzymes on stratum corneum. However, biologically active peptide and their inductors would be decomposed by the enzyme. It is desirable that enzyme, biologically active peptide and their inductors are not to be included together into the base material. Examples of said protease are pepsin, trypsin, chymotrypsin, papain, collagenase, elastase, endoproteinase, pronase etc.. Lipase may either the refined extracts from animal pancreas or the products on the market.

When said trnasdermal permeation preparation is applied to the skin, the transdermal active ingredient would permeate through the skin. However, biologically active peptide and their inductors would be decomposed by the enzyme. Therefore, when biologically active peptide and their derivatives function as the transdermal active ingredient, it is desirable to pretreat the skin with an enzyme before the transdermal permeation preparation containing no enzymen but biologically active peptide and their derivatives only to be applied to the skin. Additionally, it is also desirable that the skin is pretreated with enzyme though the transdermal active ingredient is not the biologically active peptide and their derivatives. And in such a case, the base material of the transdermal permeation preparation may contain the enzymen or not.

A typical transdermal permeation method of the transdermal permeation preparation in this invention is that the skin should be pretreated with an enzyme before the transdermal permeation preparation being applied to the skin..

First, the skin is pretreated with enzyme. And then transdermal permeation preparation is applied to the skin. With regard to skin pretreatment, it is desirable that an enzyme water solution prepared by dissolving the enzyme in purified water with a suitable pH is applied to the skin site where a transdermal permeation preparation will be applied on. It is really a simple practice to dip the filter paper or some other porous material into the enzyme aqueous solution and then to apply it to the skin site. As for the other pretreatment of skin with enzyme, the enzyme should be dissolved in an ointment matrix to prepare an enzyme ointment before being applied to the skin, or dissolved in hydrogel to prepare an enzyme hydrogel before being applied to the skin.

To obtain the effective treatment, the skin which pretreated with enzyme solution or enzyme ointment should be covered by water inpermeable membrane for 5-60 min. Then the site of pretreated skin is cleaned and patched with a transdermal permeation preparation. The transdermal active ingredient is absorbed percutaneously into the body.

This invention is to provide a typical method for producing the transdermal permeation preparation. A transdermal active ingredient in a molecule weigh higher than 1000 Dalton is dissolved or suspended in water and/or water-containing alcohol to form an aqueous solution or suspension. The aqueous solution or suspension is applied onto the surface of a base material of hydrophilic macromolecule which can swell after absorbing water and/or water-containing alcohol to cause the water or the water-containing alcohol to be absorbed in the base material, thereby forming a transderml active agent layer containing the transdermal active ingredient in a concentrated state on the surface of the base material.

With regard to this invention, firstly the transdermal active ingredient is dissolved or suspended in water or water-containing alcohol to prepare an aqueous solution or suspension. Water or water- containing alcohol can be water, water-containing methanol, water-containing ethanol etc.. Their buffer solution may also be used. Some buffer solutions as phosphate buffer saline (PBS) containing no Ca²⁺/Mg²⁺, normal salt water (150mMNaCl water solution), or tris buffer solution etc. can be used.

The transdermal active ingredeint can be dissolved in water, or water-containing alcohol, or the buffer solution to prepare an expected solution if the transdermal active ingredient can be dissolved in these solvents. If the transdermal active ingredient can not be dissolved in these solvents, it have to be dissolved in 10mM acetic acid first to prepare a solution, and then this solution is diluted with neutral water or aqueous alcohol or buffer solution to expected volume. As far as an antigen which can only be dissolved in acidic pH is concerned, the antigen should be dissolved in diluted acidic acetic first to form a solution, and then this solution is diluted by acetic acid-PBS solution in acidic pH to expected volume. Additionally, with regard to media insoluble transdermal active ingredient, it may also be suspended by surfactant.

Then prepared aqueous solution or suspension is put on the surface of said base material to cause the water or the water-containing alcohol to be absorbed into the base material. Because said base material is a hydrophilic macromolecule which can swell after absorbing water and/or water-containing alcohol, water and/or water-containing alcohol in aqueous solution or suspension can be easily absorbed into the base material (hydrophilic macromolecule), thereby forming transderml active agent layer containing the transdermal active ingredient in a concentrated state on the surface of the base material.

### Effect of the invention

The composition of the transdermal permeation preparation in this invention is as mentioned above. Because a highly concentrated transdermal active ingredient layer is laminated on surface of the base material and the concentrated transdermal active ingredient layer is to be applied to skin directly, the transdermal permeation preparation exhibit a high percutaneopus permeation effect. Additionally, the method for producing the transdermal permeation preparation in this invention is as mentioned above. It is possible to produce a transdermal permeation preparation which comprises concentrated transdermal active ingredient laminated on surface of the base material with ease. Moreover, the method of the transdermal permeation using the transdermal permeation preparation in this invention is as mentioned above. It exhibits an excellent transdermal absorptivity of the transdermal active ingredient.

Next are some of the embodiments concerning this invention.

### Skin permeation experiment

The excised rat skin was clamped on the Franz diffusion cell with water bath maintained at 37°C. The receptor cell was filled with a binary vehicle of PEG 400/water=30:70 by weight and stirred with a magnetic stirrer. The transdermal permeation preparation prepared above was mounted on the skin on the donor side. At the time interval of 24 hours, sample was removed from the receptor compartment and analyzed by HPLC method to calculate the accumulated permeation amount.

### (Example 1)

A 500ml flask was filled with 200g ethyl acetate, 43g methoxyethyl acrylate, 36g lauryl acrylate, 6g vinylpyrrolidone, 3g acrylic acid, 10g hydroxyethyl acrylate and 0.005g azobisisbutyronitrile to form a polymerization solution. The solution was heated to 75°C for 15 hours in a nitrogen atmosphere, resulting in desired copolymer.

100g of copolymer solution obtained above containing 100g of solid, 3g glycerin, 10g octyldodecyl lactate and 0.02g sodium hyaluronate were mixed completely to prepare a solution. Said solution was coated onto the PET film using a knife coater, and then dried at a temperature of 50°C for 24 hours to form a base material sheet with a thickness of 200µm. Then said sheet was punched to obtain a circular patch shaped base material in a diameter of 1cm.

A bovine insulin solution was prepared by dissolving 1000µg of bovine insulin (Nacalai Tesque Inc.) in a molecular weight of 5,733 Dalton into 1ml of phosphate buffer saline (PBS) solution containing no Ca²⁺/Mg²⁺. 100µl of said bovine insulin solution is dropped onto the base material to prepare an insulin patch which had an transdermal active ingredient layer containing 100µg bovine insulin on surface of the base material after the PBS was absorbed in the base material. Then the stratum corneum side of the rat skin was tightly in contact with wet gauze soaked with 0.01weight% of trypsin (Nacalai Tesque Inc.) solution at 36 C° for 1 hour for pretreatment of the stratum corneum. Then the skin was washed with refined water. The remaining water was wiped with gauze. And then an insulin patch prepared above was pasted on the skin to test the transdermal absorptivity of the transdermal active ingredient. The result indicated that the accumulated permeation amount within 24 hours for bovine insulin was 3.2 µg/cm².

### (Example 2)

A transdermal permeation experiment with no pretreatment of stratum corneum was also carried out using the same method as Example 1. The result indicated that the accumulated permeation amount within 24 hours for bovine insulin was 1.7 µg/cm².

### (Control 1)

1g of copolymer solution obtained above, 0.03g glycerin, 0.10g octyldodecyl lactate, 0.0002g sodium hyaluronate and bovine insulin in a molecular weight of 5,733 Dalton were mixed completely to prepare a solution. Said solution was coated onto the PET film using a knife coater, and then dried at a temperature of 50 C° for 24 hours to form a base material sheet with a thickness of 200µm. Then said sheet was punched to obtain a circular insulin patch in a diameter of 1cm. This insulin patch was used to carry out the transdermal permeation determination using the same method as Example 1. The result indicated that the accumulated permeation amount within 24 hours for bovine insulin was 0.01 µg/cm².

### (Example 3)

40 weight% of polyvinyl alcohol was coated onto the PET film to form a sheet with a thickness of 400µm. After being frozen at -60 C° for 24 hours, the sheet was thawed to room temperature to obtain the cross-linked polyvinyl alcohol hydrogel sheet. Then said sheet was punched to obtain a circular patch base material in a diameter of 1cm.

A salmon calcitonin solution was prepared by dissolving 1000µg of salmon calcitonin (Shimadzu Inc.) in a molecular weight of 5,000 Dalton into 1ml of phosphate buffer saline (PBS) solution containing no Ca²⁺/Mg²⁺. 100µl of said salmon calcitonin solution is dropped onto the base material to prepare an insulin patch which had a transdermal active ingredient layer containing 100µg salmon calcitonin on the surface of the base material after the PBS was absorbed in the base material. Then the stratum corneum side of the rat skin was tightly in contact with wet gauze soaked with 0.1 weight% of trypsin (Nacalai Tesque Inc.) solution at 36 C° for 1 hour for pretreatment of the stratum corneum. Then the skin was washed with distilled water. The remaining water was wiped with gauze. And then the hydrogel patch was pasted onto the skin to test the transdermal absorptivity of the transdermal active ingredient. The result indicated that the accumulated permeation amount within 24 hours for salmon calcitonin was 5.2 µg/cm².

### (Control 1)

1000 µg of salmon calcitonin (Shimadzu Inc.) was added into 1ml of 40 weight% of polyvinyl alcohol to prepare a solution. Said solution was then coated onto the PET film to form a sheet of a thickness 400µm. After being frozen at -60 C° for 24 hours, the sheet was thawed to room temperature to obtain the cross-linked polyvinyl alcohol hydrogel sheet. Then said sheet was punched to obtain a salmon calcitonin patch in a diameter of 1cm. This salmon calcitonin patch was used to carry out the transdermal permeation determination using the same method as Example 2. The result indicated that the accumulated permeation amount within 24 hours for salmon calcitonin was 0.03 µg/cm².

### (Example 4)

A transdermal permeation experiment with no pretreatment of the stratum corneum was also carried out using the same method as Example 2. The result indicated that the accumulated permeation amount within 24 hours for salmon calcitonin was 2.3 µg/cm².

### Possibility for industrial usage

The transdermal permeation preparation in this invention has a transdermal active ingredient layer containing transdermal active ingredient in a highly concentrated condition on the surface of the base material. Because this high concentrated transdermal active ingredient layer is applied to skin directly, the transdermal permeation preparation exhibits a high permeation effect and is applicable in medicine.

## Claims

1. A transdermal permeation preparation which is **characterized by** the laminate of macromolecular active ingredients which have molecular weight of more than 1,000 Dalton and can dissolve or suspend in water and/or a water-containing alcohol, and hydrophilic macromolecular base materials which can absorb water or water-alcohol mixtures .

2. The said transdermal permeation preparation according to claim 1, wherein said hydrophilic macromolecule is poly-alkylalkoxy acrylate, starch-acrylic acid graft polymers, polyacrylates, polyvinylalcohol, vinyl acetate-acrylic acid copolymers, polyvinyl pyrrolidone, isobutylene-maleic acid copolymer or N-vinyl acetamides.

3. The said transdermal permeation preparation according to claim 2, wherein said hydrophilic macromolecule is a crosslinked polymer.

4. The said transdermal permeation preparation according to claim 2, wherein said macromolecule of polyalkyl-alkoxy acrylic acid comprises a copolymer, octyldodecyl lactate, glycerin and hyaluronic acid in a concentration ratio of 100: 2-15:2-10:0.01-0.1 by weight respectively.; said copolymer comprises methoxyethyl acrylate, (methyl) lauryl acrylate and polar monomer in a concentration ratio of 40-60%:30-40%:10-25% by weight.

5. The said transdermal permeation preparation according to claim 2, wherein said macromolecule of polyvinylalcohol is hydrogel made from polyvinylalcohol.

6. The said transdermal permeation preparation according to claim 1, wherein said base materials contain plasticizer.

7. The said transdermal permeation preparation according to claim 1, wherein said base materials contain chemical enhancers.

8. A transdermal permeation method which is **characterized by** a pretreatment of the skin with enzyme followed by application of said transdermal permeation preparation in claim 1 to the skin.

9. A method for producing said transdermal permeation preparation according to claim 1, wherein said transdermal active ingredient of a molecular weigh higher than 1000 Dalton is dissolved or suspended in water and/or water-containing alcohol to form an aqueous solution or suspension and the said aqueous solution or suspension is coated on the surface of base materials which are hydrophilic macromolecules which can swell after absorbing the water or e water-containing alcohol as a result of absorption of the water or the water-containing alcohol in the base material, thereby forming a layer containing the transdermal active ingredient in a concentrated state on the surface of the base material.
